# EUROPÄISCHE PATENTSCHRIFT

(11) **EP 0 529 584 B1**
(45) Veröffentlichungstag und Bekanntmachung des Hinweises auf die Patenterteilung: **10.07.1996**
(21) Anmeldenummer: 92114469.7
(22) Anmeldetag: 25.08.1992
(51) Int. Cl.: C07K 16/10, A61K 39/42, C12N 7/02, C12N 7/04, C12N 7/00, A61K 39/12

(54) **Virales Agens assoziiert mit der Mystery Swine Disease**
Viral agent associated with the Mystery Swine Disease
Agent viral associé avec la Mystery Swine Disease

(30) Priorität: 26.08.1991 US 749839; 26.02.1992 US 841692; 05.08.1992 US 921891
(43) Veröffentlichungstag der Anmeldung: 03.03.1993
(73) Patentinhaber: BOEHRINGER INGELHEIM ANIMAL HEALTH INC., St. Joseph, Missouri 64502 (US)
(72) Erfinder: Chladek Danny W., St. Joseph, Mo. 64505 (US); Gorcyca, David E., St. Joseph, Mo. 64506 (US); Harris, Louis L., St. Joseph, Mo. 64506 (US)
(74) Vertreter: Laudien, Dieter, Dr.

(56) Entgegenhaltungen:
- FR-A- 2 602 791
- PHIND Database ,PJB Publications Ltd.,Surrey ,GB Abstract number 00278268 'Dutch team isolates mystery pig ...'
- DATABASE WPIL Week 8741, Derwent Publications Ltd., London, GB; AN 87-286929
- Ä41Ü

## Beschreibung

Die Erfindung betrifft die Isolierung eines viralen Agens, das mit der "Mystery Swine Disease" assoziiert ist sowie Verfahren zur Anzucht, Verfahren zur Attenuierung des Virus und zur Impfstoffherstellung.

### STAND DER TECHNIK

Kürzlich hat eine offensichtlich neue Schweineerkrankung zu schweren Verlusten in Zuchtbeständen in den Vereinigten Staaten und in bestimmten Regionen Kanadas geführt (Keffaber, K.K., "Reproductive Failure of Unknown Etiology", American Association of Swine Practitioners Newsletter, 1:109 (1989). Eine ähnliche Erkrankung wurde auch aus bestimmten Regionen Deutschlands, den Niederlanden und auch aus dem Vereinigten Königreich und Spanien gemeldet (Wensvoort, G. et al., "Blue Ear Disease of Pigs", Vet. Rec. 128:574 (1991). Das hervorstechenste klinische Symptom dieser Erkrankung ist der Wurf von toten oder geschwächten Ferkeln zusammen mit einigen scheinbar gesunden, die sich in der Folge in einem schlechten Zustand befinden oder eine beeinträchtigte Atmung und ZNS-Symptome entwickeln und sterben. In einigen befallenen Beständen gehen bis zu 75 % aller Ferkel verloren. Folglich sind die ökonomischen Konsequenzen dieser Krankheit beträchtlich. Die Krankheit wurde mit "Mystery Swine Disease", Swine Infertility and Respiratory Syndrome (SIRS)", "Blue Ear Disease" und "Porcine Reproductive and Respiratory Syndrome" bezeichnet.

SIRS ist eine offensichtlich infektöse Krankheit, die durch reproduktives Versagen, respiratorische Erkrankungen und verschiedene klinische Befunde einschließlich Anorexie, Fieber, Dyspnoe und leichte neurologische Befunde gekennzeichnet ist. Die Krankheit kommt in allen Arten von Schweineproduktionsanlagen vor und könnte zu einer der kostenträchtigsten Erkrankungen gehören, die die Schweineindustrie heutzutage betreffen (Polson, D.P. et al. "Financial Implications of Mystery Swine Disease", Proceedings, Livestock Conservation Institute, Denver, CO, 6. Oktober 1990).

Die Infektion der Sauen kann unbemerkt verlaufen oder manifestiert sich durch ein beeinträchtigtes Allgemeinbefinden, das einen Tag oder einige wenige Tage andauert. Zum Beispiel können die Sauen die Futteraufnahme verweigern und zeigen Körpertemperaturen über oder unter normal. In der Gebärphase schließen die Krankheitsanzeichen Depression, Lethargie, Pyrexie, gelegentliches Erbrechen, Abort, Totgeburten und/oder die Austragung mummifizierter Föten ein. Der häufigste klinische Befund ist ein dramatisches Anwachsen der Zahl der totgeborenen Schweine. Die Zahl der Totgeburten kann bis zu 20 % bis 30 % aller Geburten eines infizierten Bestandes betragen. Viele der als Totgeburten ausgetragenen Föten sind aufgequollen, so daß sie offensichtlich schon 24 bis 48 Stunden tot waren.

Wie oben dargestellt, ist die Hauptkomponente von SIRS das reproduktive Versagen, das sich in Frühgeburten, späten Aborten, Geburt von geschwächten Schweinen, erhöhte Zahl von Totgeburten, mummifizierte Föten, verminderte Geburtsraten und verzögerte Rückkehr zum Östrus manifestiert. Diese klinischen Befunde werden typischerweise 4 bis 16 Wochen oder sogar länger in einem Bestand beobachtet. Totgeborene Föten in betroffenen Würfen sind oft in einem frühen Zustand der Mummifizierung, was durch eine gelbbraune Verfärbung der Haut und post-mortem Autolyse angezeigt wird. Haubenförmige Mißbilden fötaler Schädel sind einige Male festgestellt worden.

Klinische Befunde einer respiratorischen Erkrankung sind am ausgeprägtesten bei Ferkeln, die jünger als drei Wochen sind. Sie werden aber auch von Schweinen jeden Alters in infizierten Beständen berichtet. Die erkrankten Ferkel wachsen langsam, besitzen ein aufgerauhtes Haarkleid, respiratorische Schmerzen und eine erhöhte Mortalität (bis zu ungefähr 80 % Mortalität bei nichtentwöhnten Ferkeln).

Befunde in vorläufigen Studien von großen und mikroskopischen Läsionen von an SIRS erkrankten Schweinen legen nahe, daß mikroskopische Lungenläsionen wichtige klinische Anzeichen dieser Erkrankung sind. Trotz ausgeprägter respiratorischer Anzeichen sind Lungen, die nicht durch sekundäre bakterielle Infektionen kompliziert sind, entweder ausgeprägt normal oder weisen eine schwache, diffuse, bräunlich-graue Verfärbung der Lungenoberfläche auf. Mikroskopische Untersuchungen des Lungengewebes von an SIRS erkrankten Ferkeln zeigen ein charakteristisches Muster interstitieller Pneumonie (Collins, J. E. et al. "Respiratory Disease in a Swine Herd Experiencing a Reproductive Failure Syndrome", Proceedings, Minnesota Swine Conference for Veterinarians, p. 254, St. Paul, MN, 10.-18. September 1990).

Das Auftreten von SIRS oder der "Mystery Swine Disease" ist weitverbreitet in den Vereinigten Staaten und wurde aus mindestens elf Staaten berichtet. Die primär verursachenden Agentien schließen, soweit in der veröffentlichen Literatur berichtet wurde, das Encephalomyocarditis Virus (EMC), das Schweine-Influenzavirus (SIV), Mycotoxine und Chlamydia ein.

### DIE ERFINDUNG

Gewebehomogenate von Ferkeln aus mit SIRS infizierten Beständen reproduzierten durchweg die respratorischen und reproduktiven Formen von SIRS, wenn sie intranasal an gnotobiotischen Ferkeln und trächtigen Sauen inokuliert wurden. Auf diese Weise inokulierte gnotobiotische Ferkel mit entweder unfiltriertem oder filtriertem Inokulum (0,45, 0,22 oder 0,1 µm) wurden anorektisch und entwickelten mikroskopische Lungenläsionen ähnlich denen aus mit SIRS befallenen Beständen. Das gleiche Inokulum verursachte auch reproduktive Effekte, identisch mit denen aus mit SIRS infizierten Beständen. Ein virales Agens wurde aus den Gewebehomogenaten erhalten. Das virale Agens verursacht eine Krankheit das SIRS an Ferkeln und trächtigen Sauen nachbildet. Das virale Agens wurde am 18. Juli 1991 bei der American Type Culture Collection in Rockville, Maryland, USA, unter dem Aktenzeichen ATCC-VR2332 hinterlegt. Das virale Agens ist ein anspruchsvoller, nicht hämagglutierendes, umhülltes RNA Virus.

### A. ISOLIERUNG

Lungengewebe und ein kombiniertes Hirn-Milz-Leber-Nieren-Gewebe wurden aus einem infizierten Ferkel aus einem mit SIRS infizierten Bestand erhalten und separat homogenisiert. Die einzelnen Homogenate wurden mit "Modified Eagle's Medium" (MEM), das ungefähr 100 µg/ml Gentamycin enthielt, gemischt. Beide Proben wurden für ca. 25 Minuten bei ungefähr 4000 x g zentrifugiert. Die Überstände wurden abgenommen und durch ein 0,45 micron Filter filtriert. Das Gewebe- und das Lungenhomogenat wurden dann vereinigt. Das vereinigte Material wurde zur Infektion verschiedener Gewebekulturflaschen verwendet.

Zwei Tests wurden in 75 cm²-Plastikflaschen durchgeführt. In Versuch Nummer 1 wurde das vereinigte Material in zwei Flaschen mit vollständiger Zellschicht aus jeder unten aufgeführten Zellinie inokuliert. Zusätzlich wurde eine Flasche jeder Zellinie mit ungefähr 2,5 mg Trypsin verstetzt. Alle verbleibenden Bedingungen waren für jede Flasche einer Zellinie gleich. Serum wurde nicht zu dem Kulturmedium gegeben. Das Inokulum betrug ungefähr 1 ml. Alle Flaschen wurden für ungefähr sieben Tage bei annähernd 4°C gehalten. Die Resultate wurden am Ende von ungefähr sieben Tagen aufgenommen. Nach Einfrieren und Auftauen wurde eine Probe für eine zweite Passage in der gleichen Zellinie abgenommen. Das restliche Material wurde eingefroren und bei -60°C aufbewahrt.

In Versuch Nummer 2 wurde das vereinigte Material in Flaschen inokuliert, die die gleichen Zellinien enthielten, die auch in Versuch Nummer 1 verwendet wurden. Jedoch waren die Zellschichten zum Zeitpunkt der Inokulation nur zu 20 bis 40 % konfluent. Die Medien enthielten 10 % fötales Kälberserum. Wieder war das Inokulum ungefähr 1 ml und die Kulturen wurden für annähernd 7 Tage bei ungefähr 34°C inkubiert. Das Resultat der beiden Versuche ist unten zusammengefaßt.

| benutzte Zellinie | Test Nummer 1 | Test Nummer 2 |
|---|---|---|
| bovine Nasenmuschel (BT) | - | - |
| Katzenniere (CRFK) | - | - |
| Affenniere (VERO) | - | - |
| Affenlunge (VERO) | - | - |
| Kaninchenniere (MDCK) | - | - |
| Schweineniere (PK2a) | - | - |
| Nerzlunge | - | - |
| Frettchenlunge | - | - |
| Rinderlunge | - | - |
| Büffellunge | - | - |
| Rinderniere (MDBK) | - | - |
| Schweinehoden (ST) | - | - |
| Affenniere (MA-104) | - | + |
| humaner rektaler Tumor (HRT-18) | - | n.b. |
| humane Lunge | n.b. | - |
| + = cytopathischer Effekt (CPE) | | |
| - = kein cytopathischer Effekt | | |
| n.b. = nicht bestimmt | | |

In Test Nummer 1 wurde in allen Zellinien kein cytopathischer Effekt beobachtet. Jedoch begannen in Test Nummer 2 kleine Klumpen aus MA-104-Zellen anzuschwellen und "schwache Löcher" in der Einzelschicht in den Flaschenecken zu bilden. Flüssigkeit wurde aus der Flasche entnommen und in eine neue Flasche mit MA-104 Zellen gegeben (wieder mit 20-40 % Zellschicht); dies wurde ein drittes Mal wiederholt. Der cytopathische Effekt wurde mit jeder Passage größer. Die oben beschriebenen Verfahren wurden für die MA-104 Zellinie mit einer vollständigen Zellschicht wiederholt. Ein cytopathischer Effekt (CPE) wurde ebenfalls beobachtet.

Weitere Versuche zeigten, daß das Virus auch bei ungefähr 37°C wächst. Die Anwesenheit von Serum könnte hilfreicher für eine erste Isolation des viralen Agens sein. Nachfolgende Passagen des viralen Agens in der MA-104 Zellinie zeigen einen CPE ohne Zugabe von Serum. Jedoch wird ein weitaus ausgeprägter CPE unter Verwendung von Serum im Wachstumsmedium für die MA-104 Zellinie beobachtet.

Es wurde auch nachgewiesen, daß das virale Agens teilweise hitzebeständig bei 56°C für annähernd 50 Minuten ist. Als Ergebnis einer solchen Hitzebehandlung wurde der Virustiter um ungefähr drei Zehnerpotenzen herabgesetzt. Das virale Agens wurde achtmal einer Passage mit gutem CPE, der sich nach drei Tagen in Passage fünf und größer entwickelte, unterworfen. Der erhaltene Titer betrug annähernd 10^{5,5}. Das virale Agens wächst auch in anderen Affenzellinien. Demzufolge gehört es zum Wissen eines Fachmanns auf diesem Gebiet, das SIRS Virus ATCC-VR2332 oder davon abgeleitete Mutanten in anderen Zellinien durch Modifikation der Wachstumsbedingungen, der Wachstumsmedien, usw., anzuziehen.

### B. IN VIVO VERSUCHE

Die Ernte nach einer dritten Passage wurde für die Inokulation zweier drei Tage alter gnotobiotischer Ferkel verwendet. Beide Ferkel wurden nasal exponiert. Eines mit 1 ml und das andere mit 2 ml. Die Ferkel wurden 7 Tage beobachtet, dann wurden sie getötet.

Gewebeproben wurden für die Histopathologie und für die Wiedergewinnung des viralen Agens verwendet. Der histopathologische Befund bestätigte, daß Lungenläsionen der infizierten Ferkel mit Lungenläsionen identisch waren, die aus an SIRS erkrankten Ferkeln stammten.

Die Gewebeproben wurden wie zuvor beschrieben aufgearbeitet und dann auf einer 20 bis 40 % und 100 % Einzelschicht der MA-104 Zellinie mit fötalem Rinderserum kultiviert. Das virale Agens wurde erneut zurückgewonnen.

Die Ernte nach einer dritten Passage wurde ebenfalls für die Inokulation von Sauen verwendet, um die reproduktiven Effekte der Krankheit zu duplizieren und zu bestätigen. Zwei mehrfach trächtige Sauen wurden am ungefähr 93. Tag der Trächtigkeit intranasal inokuliert. Die Sauen wiesen Würfe mit 50 % totgeborenen Ferkeln am 112. bzw. 114. Tag der Trächtigkeit auf (8/13 und 6/14 totgeborene/lebendgeborene). Sieben der totgeborenen Ferkel waren teilweise mummifiziert, die lebendgeborenen Ferkel waren schwach und konnten nicht kräftig saugen. Das virale Agens wurde aus dem Gewebe der totgeborenen Ferkel wiedergewonnen.

Das virale Agens wurde aus drei Beständen gewonnen, von denen man wußte, das sie mit SIRS infiziert waren. Antikörpertiter gegen das ATCC-VR2332 Virus wurden in denselben Beständen nachgewiesen.

Obgleich einige Unterschiede in den klinischen Befunden auftraten, d.h. kutane Zyanose der Ohren, des Schwanzes und des Euters an europäischen Schweinen, ist die vorherrschende Meinung, das die nordamerikanische und die europäische Krankheit durch das gleiche Virus verursacht werden.

### C. VIRALE CHARAKTERISTIKA

ATCC-VR2332 verursacht durchweg klinische Befunde und pulmonale Läsionen in gnotobiotischen Schweinen sowie negative ImF Resultate, was darauf hindeutet, daß das Virus keine Gruppenantigene besitzt, die antigenisch ähnlich denen der Viren aus den gegenwärtigen Genera der Togaviridae sind. Das ATCC-2332 könnte demnach eine noch nicht identifizierte Gattung der Togaviridae darstellen. ATCC-2332 ist auch kein bekanntes Schweinepathogen, da spezifische Antisera gegen mehrere bekannte virale Schweinepathogene nicht in der Lage waren, das Virus zu neutralisieren oder Antigene in infizierten Zellen nachzuweisen.

ATCC-VR2332 ist ein anspruchsvolles, nicht hämagglutinierendes, umhülltes RNA Virus. Das ATCC-VR2332 Virus wächst in einer kontinuierlichen Zellinie, insbesondere in MA-104 und anderen Affenzellinien. Das ATCC-VR2332 Virus wächst zu einem hohen Titer (10⁷TClD₅₀/1 ml) in der kommerziell verfügbaren Zellinie MA-104 heran.

Das ATCC-VR2332 Virus besitzt eine Lipidumhüllung, was durch einen Verlust an Infektivität nach Chloroformbehandlung nachgewiesen wurde. Eine Lipidumhüllung kann auch als durchscheinender Ring um die leeren Partikel nachgewiesen werden. Die Morphologie des SIRS Virus ist in der direkten Elektronenmikroskopie (DEM) nicht ausgeprägt und nur ausgesprochen schwer in Präparationen zu identifizieren, die zelluläre Rückstände enthalten. Das ATCC-VR2332 Virus kann nach Reinigung mittels CsCl-Gradienten, gefolgt durch Immunogold-Markierung des Virions mit anti-ATCC-VR2332 Hyperimmunsera und Goldkonjugat identifiziert werden. Die Schwebedichte der ATTC-VR2332 Virionen in CsCl-Gradienten liegt bei 1,18 bis 1,19 g/ml. Sucrosegradienten weisen durchweg einen beträchtlichen Virustiterverlust auf und wurden als geeigneter Gradient für die Reinigung verworfen. Die Morphologie der Gradienten-gereinigten ATCC-VR2332-Partikel und der durchschnittliche Durchmesser von 62 mm sind ähnlich denen der Virionen der equinen Arteritis und denen der Lactat-Dehydrogenare Viren. Für equine Arteritis Viren wird ein Größenbereich von 50-73 nm angegeben, ähnlich der 48-84 nm Größe für das ATCC-VR2332-Virus. 30-35 nm Kerne in mehreren ATCC-VR2332-Partikeln wurden beobachtet, wobei die Kerne an die Nucleocapsidkerne erinnern, wie sie für das equine Arteritis Virus beschrieben wurden. Morphologisch gesehen steht das ATCC-VR2332 Virus der Arteritis Virusgruppe am nächsten.

Die Präsens eines RNA Genoms von ATTC-VR2332 wurde durch die Fähigkeit des Virus zu einer Fortsetzung der Replikation in Gegenwart von 5-Brom-2- Deoxyuridin und Mitomycin C bestätigt, die dafür bekannt sind, die Replikation der DNA und einer RNA Virusfamilie (Retroviridae) - nicht aber von anderen RNA Viren - zu inhibieren. Außerdem stimmt die vorläufige Klassifizierung des ATCC-VR2332 Virus als RNA Virus mit der Beobachtung überein, daß das Virus im Cytoplasma einer Zelle repliziert, was durch die Präsens von Virusantigenen gezeigt wird, die durch ImF nachgewiesen wurden. Auch Actinomycin D, das mit der DNA-abhängigen RNA-Transkription interferiert, hat keinen Effekt auf die Replikation des ATCC-VR2332 Virus. Diese Ergebnisse zeigen, daß das ATCC-VR2332 Virus keine nuklearen Funktionen für die Replikation benötigt.

Das ATCC-VR2332 Virus ist hitzelabil bei 37°C und 56°C, aber für lange Zeit stabil bei 4°C und -70°C. Die Thermolabilität des Virus bei 37°C legt nahe, daß das Virus relativ instabil in warmer Umgebung ist. Dies hat auch eine praktische Bedeutung für die Vermehrung des Virus, indem Wachstumstemperaturen geringer als 37°C höhere Ausbeuten an ATCC-VR2332-Virus liefern. Kühlung sollte für das kurzzeitige Aufbewahren diagnostischer Proben für die Virusisolation genügen, andernfalls sollte die Probe eingefroren werden.

Zusammengefaßt lassen Größe, Morphologie, Anwesenheit eines RNA-Genoms und andere biologische Eigenschaften das ATCC-VR2332 Virus vorläufig der Familie der Togaviridae zugehörig erscheinen. ATCC-VR2332 kann jedoch nicht definitiv in eine Gattung dieser Familie eingeordnet werden. Obgleich das ATCC-VR2332 Virus morphologisch gesehen mit den Arteritis Viren nahe verwandt erscheint, sollte das Virus solange nicht in eine definierte Gattung eingeordnet werden, solange nicht zusätzliche Information zur RNA- und Proteinstruktur verfügbar sind.

### D. MODIFIZIERTE LEBEND-IMPFSTOFF-HERSTELLUNG

Eine Impfstoffherstellung wurde formuliert durch Einschluß eines modifizierten oder eines attenuierten lebenden ATCC-VR2332, dessen Verabreichung Schweine erfolgreich gegen eine Infektion immunisierte. Das SIRS Virus ATCC-VR2332 wurde in der kontinierlichen MA-104 Zellinie propagiert. Die Zellinie wurde in Flaschen angezogen, die MEM enthielten, zu dem 10 % fötales Kälberserum zugegeben worden war. Der pH-Wert des Mediums wurde auf 7,2 eingestellt und das Medium bei annähernd 37°C inkubiert. Das virus wurde durch Zugabe von ungefähr 1 ml eines eingefrorenen Inokulums zu dem flüssigen Medium in die Zellen inokuliert. Anschließend ließ man das Virus für 24 Stunden an die Zellen absorbieren.

Dann wurde das Wachstumsmedium durch ein Erhaltungsmedium ersetzt, das aus MEM bestand, zu dem 4 % fötales Kälberserum, pH 7.6, zugegeben worden waren. Die umgebungstemperatur betrug 35-37°C. Das Virus wurde solange angezogen, bis 50 t der MA-104 Zellschicht durch das Virus zerstört waren. Die Probe wurde eingefroren und für eine Passage in einer anderen Flasche mit MA-104 Zellen verwendet. Dieser Prozeß wurde für 25 Passagen des Virus in der Zellinie durchgeführt. Das Virus wurde anschließend weitere 12 Mal bei circa 31°C propagiert, im Gegensatz zu den 35 bis 37°C, wobei aber die gleichen Techniken wie oben beschrieben verwendet wurden. Die zwölfte Passage wurde in Form kleiner Aliquots eingefroren und als "Master Seed Virus" bezeichnet.

Im einzelnen umfaßt die Erfindung demnach folgende Gegenstände
- ein Verfahren zur Attenuierung des SIRS Virus, ATCC-VR2332, dadurch gekennzeichnet, daß es die Passage des Virus über eine Affenzellinie bei ungefähr 35 bis 37°C und anschließend die Passage des resultierenden Virus über eine Affenzellinie bei ungefähr 31°C umfaßt,
- ein Verfahren zur Attenuierung wie oben beschrieben, dadurch gekennzeichnet, das die Passagen über eine Affenzellinie in Erhaltungsmedium in Gegenwart von Serum bei einem pH von ungefähr 7.6 durchgeführt wird,
- ein Verfahren zur Attenuierung wie oben beschrieben, dadurch gekennzeichnet, daß die Passage bei ungefähr 34-37°C fünfundzwanzigmal und die Passage bei 31°C zwölfmal durchgeführt wird,
- ein Verfahren zur Attenuierung wie oben beschrieben, dadurch gekennzeichnet, daß die Affenzellinie MA-104 ist,
- ein Verfahren zur Attenuierung wie oben beschrieben, dadurch gekennzeichnet, daß die Passagen ohne CO₂ durchgeführt werden,
- eine Impfstoffzusammensetzung, herstellbar gemäß einen der ober beschriebenen Verfahren,
- eine Verwendung der Impfstoffzusammensetzung zur Herstellung eines Arzneimittels zur Immunisierung von Schweinen gegen SIRS.

### BEISPIEL

### Beispiel für eine Präparation von attenuiertem ATCC-VR233

### I. Medien:

a. "Eagles Minimum Essential Medium (MEM) von JRH Biosciences, # 200-2041
b. Fötales Kälberserum (FCS) von JRH Biosciences
c. Wachstumsmedium für Zellanzucht, MEM und 10 % Fötales kälberserum
d. Erhaltungsmedium: MEM, 4 % fötales Kälberserum
e. Trypsin-Versene IX
f. Natriumbicarbonat, 5 % oder gesättigt

### II. Gewebekultur:

benutzte Zellinie: MA-104 ("African Green Monkey Kidney"-Zellen, die innerhalb einer Grenze von 20 Zellpassagen gehalten werden (Zellpassage 58-78).

### III. Ausrüstung:

75 cm Gewebekulturflaschen
Inkubator bei 35-37°C
Inkubator bei 31°C
Zentrifuge

### IV. Methode zur Herstellung von attenuiertem SIRS VR-2332-Virus angezogen bei 35-37°C

### A. Herstellung einer Gewebestammkultur:

1. 5 bis 7 Tage alte MA-104 75 cm²-Stammflaschen wurden auf folgende Weise 1:4 aufgeteilt:
   a. Abgießen des gesamten Mediums (50 ml pro Flasche)
   b. Entfernen der Zellschicht mittels 10 ml Trypsin-Versene durch Inkubation bei 37°C für 5 bis 10 Minuten
   c. Entnahme der Zellen aus der Flasche und Zentrifugation bei 270 x g für 5 bis 10 Minuten
   d. Dekantieren des Überstandes und Resuspendieren der Zellen in 5-10 ml Wachstumsmedium (MEM, 10 % FCS).
   e. Aufnahme aller Zellen in 200 ml MEM, 10 % FCS, das dann 1:4 auf vier 75 cm-Flaschen, 50 ml pro Flasche, suspendiert und aufgeteilt wird. Die Flaschen werden dann bis 35-37°C aufbewahrt bei sie bebraucht werden (kann auch ohne CO₂ durchgeführt werden).
   f. 3 oder 4 Tage alte Flaschen, die eine vollstände Zellschicht gebildet haben, sind nun fertig zum Gebrauch.
   g. Einstellen des pH-Wertes von 50 ml Medium in Flaschen auf pH 7,2 and anschließende Zugabe von 1 ml SIRS Virus zum Medium und Inkubation der Flaschen bei 35-37°C (kann ohne CO₂ durchgeführt werden).
   h) Nach 24 Stunden wird das Medium verworfen und die Flaschen mit 50 ml MEM, 4 % FCS, pH 7,6 aufgefüllt und wieder bei 35-37°C inkubiert.
   i) 24 Stunden nach dem Flüssigkeitswechsel sollte sich ein cytopathischer Effekt zeigen; wenn 50-60 % der Zellschicht durchlöchert sind, werden die Flaschen eingefroren.
   j) Nach Auftauen oben beschriebener Flasche kann 1 ml dieser Flüssigkeit aufgenommen werden, in eine neue 75 cm-Flasche wie oben beschrieben überführt werden, um eine neue Zellpassage des SIRS VR-2332-Virus durchzuführen.

Das oben beschriebene Verfahren wurde insgesamt 25 Mal durchgeführt bzw. das SIRS VR-2332 wurde 25 Mal bei 35 bis 37°C einer Zellpassage unterworfen.

### V. Verfahren zur Herstellung von attenuiertem SIRS VR-2332-Virus, angezogen bei 31°C

(Es ist wichtig festzuhalten, daß das VR-2332-Virus das einer 25-fachen Zellpassage bei 35-37°C unterworfen wurde, benutzt wurde um die erste Zellpassage für die SIRS VR-2332-Virusanzucht bei 31°C durchzuführen)

### A. Präparation der Gewebestammkultur:

1. 5 bis 7 Tage alte MA-104 75 cm-Stammflaschen wurden auf folgende Weise 1:4 aufgeteilt:
   a. Abgießen des gesamten Mediums (50 ml pro Flasche)
   b. Entfernen der Zellschicht mittels 10 ml Trypsin-Versene durch Inkubation bei 37°C für 5 bis 10 Minuten
   c. Entnahme der Zellen aus der Flasche und Zentrifugation bei 270 x g für 5 bis 10 Minuten
   d. Dekantieren des Überstandes und Resuspendieren der Zellen in 5-10 ml Wachstumsmedium (MEM, 10 % FCS).
   e. Aufnahme aller Zellen in 200 ml MEM, 10 % FCS, das dann 1:4 in vier 75 cm-Flaschen, 50 ml pro Flasche, suspendiert und aufgeteilt wird. Die Flaschen werden dann bei 35-37°C aufbewahrt bis sie bebraucht werden (kann auch ohne CO₂ durchgeführt werden).
   f. 3 oder 4 Tage alte Flaschen, die eine vollstände Zellschicht gebildet haben, sind nun fertig zum Gebrauch.
   g. Einstellen des pH-Wertes von 50 ml Medium in Flaschen auf pH 7,2 and anschließende Zugabe von 1 ml SIRS Virus zum Medium und Inkubation der Flaschen bei 31°C (kann ohne CO₂ durchgeführt werden).
   h) Nach 24 Stunden wird das Medium verworfen und die Flaschen mit 50 ml MEM, 4 % FCS, pH 7,6 aufgefüllt und wieder bei 31°C inkubiert.
   i) 24 Stunden nach dem Flüssigkeitswechsel sollte sich ein cytopathischer Effekt zeigen; wenn 50-60 % der Zellschicht durchlöschert sind, werden die Flschen eingefroren.
   j) Nach Auftauen oben beschriebener Flasche kann 1 ml dieser Flüssigkeit aufgenommen werden, in eine neue 75 cm-Flasche wie oben beschrieben überführt werden, um eine neue Zellpassage des SIRS VR-2332-Virus durchzuführen.

Die obige Prozedur wurde insgesamt zwölfmal bei 31°C durchgeführt. Die zwölfte Passage des bei 31°C angezogenen Virus wurde als "Master Seed Virus" für die Impfstoffproduktion bezeichnet.

Das kalt adaptierte Virus ("Master Seed Virus") mit einem Titer von annähernd 10^{4,5} bis ungefähr 10^{5,0} TClD₅₀ pro Milliliter wurde gewöhnlichen Schweinen intranasal und intramuskulär verabreicht. Ein pharmazeutischer Trägerstoff wurde gewöhnlichen Schweinen als Kontrolle intranasal und intramuskulär verabreicht. Die mit dem attenuierten Virus geimpften Schweine entwickelten, nachdem sie dem ATCC-VR2332 Virus (Titer: 10^{4,2} TClD₅₀ pro Milliliter) ausgesetzt waren, keine SIRS-Symptome. Der kalt-adaptierte Virus kann mit jedem kompatiblen, konventionellen pharmazeutischen Trägermaterial kombiniert werden. Obwohl die Impfstoffpräparation intranasal und intramuskulär verabreicht wurde, sind natürlich auch andere Verabreichungsformen möglich und vorstellbar.

Für den Fachmann ist es natürlich offensichtlich, daß verschiedene Modifikationen der Wachstumsbedingungen, verschiedene Methoden der Attenuierung des SIRS Virus ATCC-VR2332, der Präparation von Impfstoffformulierungen ohne weiteres gemacht und abgeleitet werden können.

## Patentansprüche

1. Verfahren zur Attenuierung des SIRS Virus, ATCC-VR2332, dadurch gekennzeichnet, daß es Passagen des Virus in einer Affenzellinie bei ungefähr 35 bis 37°C und anschließend Passagen des resultierenden Virus in einer Affenzellinie bei ungefähr 31°C umfaßt.

2. Verfahren gemäß Anspruch 1, dadurch gekennzeichnet, daß die Passagen in einer Affenzellinie in Erhaltungsmedium in Gegenwart von Serum bei einem pH von ungefähr 7.6 durchgeführt werden.

3. Verfahren gemäß Anspruch 1 oder 2, dadurch gekennzeichnet, daß die Passage bei ungefähr 34-37°C fünfundzwanzigmal und die Passage bei 31°C zwölfmal durchgeführt wird.

4. Verfahren gemäß einem der Ansprüche 1 bis 3, dadurch gekennzeichnet, daß die Affenzellinie MA-104 ist.

5. Verfahren gemäß Anspruch 4, dadurch gekennzeichnet, daß die Passagen ohne CO₂ durchgeführt werden.

6. Impfstoffzusammensetzung herstellbar gemäß einem der Ansprüche 1 bis 5.

7. Verwendung der Impfstoffzusammensetzung gemäß Anspruch 6 zur Herstellung eines Arzneimittels zur Immunisierung von Schweinen gegen SIRS.

## Claims

1. Process for attenuating the SIRS virus, ATCC-VR2332, characterised in that it comprises passages of the virus in a monkey cell line at about 35 to 37°C and then passages of the resulting virus in a monkey cell line at about 31°C.

2. Process according to claim 1, characterised in that the passages are carried out in a monkey cell line in maintenance medium in the presence of serum at a pH of about 7.6.

3. Process according to claim 1 or 2, characterised in that the passage at about 34-37°C is carried out twenty-five times and the passage at 31°C is carried out twelve times.

4. Process according to one of claims 1 to 3, characterised in that the monkey cell line is MA-104.

5. Process according to claim 4, characterised in that the passages are carried out without CO₂.

6. Vaccine composition which may be prepared according to one of claims 1 to 5.

7. Use of the vaccine composition according to claim 6 for preparing a pharmaceutical composition for immunising pigs against SIRS.

## Revendications

1. Procédé pour atténuer le virus du SIRS, ATCC-VR2332, caractérisé en ce qu'il comprend des passages du virus dans une lignée cellulaire simienne à environ 35 à 37°C puis des passages du virus résultant dans une lignée cellulaire simienne à environ 31°C.

2. Procédé selon la revendication 1, caractérisé en ce que les passages dans une lignée cellulaire simienne sont réalisés dans du milieu de conservation en présence de sérum à un pH d'environ 7,6.

3. Procédé selon la revendication 1 ou 2, caractérisé en ce que le passage à environ 34-37°C est réalisé vingt cinq fois et le passage à 31°C est réalisé douze fois.

4. Procédé selon l'une des revendications 1 à 3, caractérisé en ce que la lignée cellulaire simienne est MA-104.

5. Procédé selon la revendication 4, caractérisé en ce que les passages sont réalisés sans CO₂.

6. Composition de vaccin qui peut être préparée selon l'une des revendications 1 à 5.

7. Utilisation de la composition de vaccin selon la revendication 6 pour la préparation d'un médicament pour l'immunisation des porcs contre le SIRS.
